# EUROPEAN PATENT SPECIFICATION

(11) **EP 1 770 088 B1**
(45) Date of publication and mention of the grant of the patent: **11.11.2009**
(21) Application number: 06019380.2
(22) Date of filing: 15.09.2006
(51) Int. Cl.: C07D 307/48

(54) **Process for preparing 5-methyl-2-furfural**
Verfahren zur Herstellung von 5-methyl-2-furfural
Procédé de preparation de 5-methyl-2-furfural

(30) Priority: 30.09.2005 JP 2005286834
(43) Date of publication of application: 04.04.2007
(73) Proprietor: Sumitomo Chemical Company, Limited, Tokyo 104-8260 (JP)
(72) Inventor: Hirota, Masaji, Niihama-shi Ehime-ken (JP)
(74) Representative: Vossius & Partner

(56) References cited:
- JP-A- 2002 255 951

## Description

The present invention relates to a novel process for preparing 5-methyl-2-furfural which is useful as an intermediate in the production of agrochemicals, and the like.

For preparing 5-methyl-2-furfural, a process comprising the steps of reacting 2-methylfuran, phosgene and N,N-dimethylformamide, and then hydrolyzing the reaction mixture is known. In the above reaction, usually, an organic solvent is used as a reaction medium. For example, JP-A-57-91982 discloses the use of aliphatic hydrocarbons, aromatic hydrocarbons, chlorinated aliphatic hydrocarbons, chlorinated aromatic hydrocarbons, acetonitrile or ethers as reaction media, and describes specific examples using 1,2-dichloroethane or chloroform. JP-A-2002-255951 discloses the use of aliphatic hydrocarbons, aromatic hydrocarbons, chlorinated aliphatic hydrocarbons, chlorinated aromatic hydrocarbons or ethers as reaction media, and describes a specific example using chlorobenzene. Furthermore, JP-A-2003-183268 discloses the use of halogenated aromatic hydrocarbons, and describes a specific example using chlorobenzene and also one using 1,2-dichloroethane.

When the halogenated hydrocarbons are used as reaction media as disclosed in the above patent publications, proper attention to environment is necessary. This leads to the increase of the load and costs of operations in relation to the treatment of waste water or waste gas. When the ethers are used as reaction media, attention to accident prevention is necessary. This also leads to the increase of the load and costs of operation. When acetonitrile is used as a reaction medium, because of its solubility in water, a mixture obtained after hydrolysis would not be separated into an oil phase and an aqueous phase but forms a homogeneous phase so that extra effort is necessary to isolate 5-methyl-2-furfural.

When the hydrocarbons are used, the above problems caused by other solvents may be solved. However, an intermediate immonium salt, i.e., 5-methyl-2-furyl-CH=N⁺(CH₃)₂Cl⁻, which is generated by the reaction of 2-methylfuran with a so-called Vilsmeier complex (ClHC=N⁺(CH₃)₂Cl⁻) formed from phosgene and N,N-dimethylamide, easily precipitates, and thus scaling forms or a mixed state deteriorates, so that the handling property and the yield of the product tend to decrease.

An object of the present invention is to provide a process for preparing 5-methyl-2-furfural in a high yield from 2-methylfuran, phosgene and N,N-dimethylformamide with good operability.

It has been found that the above object can be achieved by carrying out the reaction of 2-methylfuran, phosgene and N,N-dimethylformamide in the presence of a hydrocarbon as a reaction medium in a specific reaction temperature range.

Accordingly, the present invention provides a process for preparing 5-methyl-2-furfural comprising the steps of reacting 2-methylfuran, phosgene and N,N-dimethylformamide in a reaction medium comprising a hydrocarbon at a temperature of at least 55°C and hydrolyzing a reaction mixture.

In the process of the present invention, a hydrocarbon is used as a reaction medium when 2-methylfuran, phosgene and N,N-dimethylformamide are reacted, in particular, when 2-methylfuran is reacted with the Vilsmeier complex formed from phosgene and N,N-dimethylformamide to obtain the immonium salt which is a precursor of 5-methyl-2-furfural.

Examples of the hydrocarbon include aliphatic hydrocarbons having 6 to 12 carbon atoms, preferably 6 to 8 carbon atoms (e.g. hexane, heptane, isooctane, etc.), alicyclic hydrocarbons having 5 to 8 carbon atoms, preferably 5 or 6 carbon atoms (e.g. cyclopentane, cyclohexane, etc.) and aromatic hydrocarbons having 6 to 12 carbon atoms, preferably 6 to 8 carbon atoms (e.g. benzene, toluene, xylene, ethylbenzene, etc.). These hydrocarbons may be used singly or as a mixture thereof. Among them, aromatic hydrocarbons are preferably used.

The amount of the hydrocarbon is usually 1 to 50 parts by weight, preferably 2 to 10 parts by weight, per one part by weight of 2-methylfuran.

Optionally, a solvent other than the hydrocarbon may be used in combination with the hydrocarbon. In such a case, the amount of other solvent may be 0.1 part by weight or less per one part by weight of the hydrocarbon.

The amount of phosgene is usually at least one mole, preferably 1 to 1.2 moles, per one mole of 2-methylfuran. The amount of N,N-dimethylamide is also at least one mole, preferably 1 to 1.2 moles, per one mole of 2-methylfuran. Phosgene may be used in the gas state or the liquid state, or in the form of a solution in the hydrocarbon used as the reaction medium or other solvent.

In the process of the present invention, the reaction temperature is at least 55°C, when 2-methylfuran, phosgene and N,N-dimethylformamide are reacted. When the reaction temperature is at least 55°C, the precipitation of the immonium salt is suppressed so that the reaction is carried out with good operability, and thus 5-methyl-2-furfural can be obtained in a high yield after hydrolysis of the reaction mixture, although the hydrocarbon is used as the reaction medium. The reaction temperature is preferably not higher than about 100°C, since the yield of 5-methyl-2-furfural may decrease at an excessively high reaction temperature.

A mixing procedure of 2-methylfuran, phosgene, N,N-dimethylformamide and the hydrocarbon is not restricted. Preferably, the reaction medium comprising the hydrocarbon is firstly charged in a reactor, and then 2-methylfuran, phosgene and N,N-dimethylformamide are concurrently fed thereto (co-feeding) from the viewpoint of the suppression of side reactions.

The reaction in the process of the present invention may be carried out under atmospheric pressure or a reduced or elevated pressure. The progress of the reaction may be monitored with a conventional method such as gas chromatography, high-performance liquid chromatography, thin layer chromatography, NMR spectrum, etc.

The reaction mixture from the previous reaction is then hydrolyzed by mixing it with water, preferably a basic aqueous solution to convert the immonium salt in the reaction mixture to 5-methyl-2-furfural.

The amount of water to be used in the hydrolysis step is selected so that the amount of water is sufficient for hydrolyzing the immonium salt and the possibly remaining Vilsmeier complex, and a mixture having good oil-water separability is obtained.

The hydrolysis temperature is usually from about 20 to about 100°C. At least in the initial stage of the hydrolysis, the temperature is preferably at least 45°C as in the case of the reaction of 2-methylfuran, phosgene and N,N-dimethylformamide, since the precipitation of the immonium salt can be suppressed.

After the hydrolysis reaction, the reaction mixture is separated into an oil phase containing 5-methyl-2-furfural and an aqueous phase. Then, the oil phase may be purified by washing, distillation, etc., if necessary. The product can be used in various applications such as perfume compounds, agrochemicals and medicinal drugs.

The present invention will be illustrated by the following examples, which do not limit the scope of the present invention in any way. In the examples, "%" is "% by weight" unless otherwise indicated.

### EXAMPLES

Example 1

In a four-necked 2 liter flask equipped with a condenser tube, toluene (798 g) was charged and heated to 60°C while stirring. In toluene, 2-methylfuran (165 g) was added at the same temperature over 11 hours. At the same time as the start of the addition of 2-methylfuran, the addition of N,N-dimethylformamide and phosgene was started, and N,N-dimethylformamide (155 g) and phosgene (215 g) were added over 12 hours. After the completion of the addition of these compounds, the mixture was kept standing at 60°C for 1 hour. Then, water (285 g) was added while maintaining the mixture at 45°C. Up to this step, no precipitation of any solid material was observed.

To the oil-water mixture from the previous step, a 25% aqueous solution of sodium hydroxide (395 g) was added at 25°C to adjust the pHto 8. 9, and the mixture was kept standing for 1 hour. Then, the mixture was separated into an organic phase and an aqueous phase, and the organic phase was washed three times with 4% hydrochloric acid (each 125 g). After washing, the organic phase (983 g) was analyzed by gas chromatography. The content of 5-methyl-2-furfural was 20.7% (203.6 g, yield: 92%). The aqueous phase and the hydrochloric acid used for washing the organic phase contained 9.5 g of 5-methyl-2-furfural in total. When this amount of 5-methyl-2-furfural was combined with the amount of 5-methyl-2-furfural in the organic phase, the yield of 5-methyl-2-furfural was 96%.

Example 2

In a four-necked 500 milliliter flask equipped with a condenser tube, toluene (252 g) was charged and heated to 50°C while stirring. In toluene, 2-methylfuran (66 g) was added at the same temperature over 3.5 hours. At the same time as the start of the addition of 2-methylfuran, the addition of N,N-dimethylformamide and phosgene was started, and N,N-dimethylformamide (62 g) and phosgene (91 g) were added over 4 hours. After the completion of the addition of these compounds, the mixture was kept standing at 50°C for 1 hour. Then, water (114 g) was added while maintaining the mixture at 45°C. Up to this step, no precipitation of any solid material was observed.

To the oil-water mixture from the previous step, a 25% aqueous solution of sodium hydroxide (211 g) was added at 25°Cto adjust the pH to 8.9, and the mixture was kept standing for 1 hour. Then, the mixture was separated into an organic phase and an aqueous phase, and the organic phase was washed three times with a 5% aqueous solution of sodium sulfate (each 181 g). After washing, the organic phase (286 g) was analyzed by gas chromatography. The content of 5-methyl-2-furfural was 24.6% (70.3 g, yield: 79%). The aqueous phase and the aqueous solution of sodium sulfate used for washing the organic phase contained 12.4 g of 5-methyl-2-furfural in total. When this amount of 5-methyl-2-furfural was combined with the amount of 5-methyl-2-furfural in the organic phase, the yield of 5-methyl-2-furfural was 93%.

Comparative Example 1

In a four-necked 500 milliliter flask equipped with a condenser tube, toluene (252 g) was charged and heated to 40°C while stirring. In toluene, 2-methylfuran (66 g) was added at the same temperature over 3.5 hours. At the same time as the start of the addition of 2-methylfuran, the addition of N,N-dimethylformamide and phosgene was started, and N,N-dimethylformamide (62 g) and phosgene (91 g) were added over 4 hours. After 2.8 hours from the start of the addition of the compounds, the solid started to precipitate and thus the stirring of the reaction mixture became difficult. After the completion of the addition of the compounds, the mixture was kept standing at 40°C for 1 hour. Then, water (114 g) was added while maintaining the mixture at 40°C. The solid was dissolved by the addition of water.

To the oil-water mixture from the previous step, a 25% aqueous solution of sodium hydroxide (166 g) was added at 25°C to adjust the pH to 8.9, and the mixture was kept standing for 1 hour. Then, the mixture was separated into an organic phase and an aqueous phase, and the organic phase was washed three times with a 5% aqueous solution of sodium sulfate (each 181 g). After washing, the organic phase (300 g) was analyzed by gas chromatography. The content of 5-methyl-2-furfural was 24.4% (73.6 g, yield: 83%). The aqueous phase and the aqueous solution of sodium sulfate used for washing the organic phase contained 7.8 g of 5-methyl-2-furfural in total. When this amount of 5-methyl-2-furfural was combined with the amount of 5-methyl-2-furfural in the organic phase, the yield of 5-methyl-2-furfural was 92%.

## Claims

1. A process for preparing 5-methyl-2-furfural comprising the steps of reacting 2-methylfuran, phosgene and N,N-dimethylformamide in a reaction medium comprising a hydrocarbon at a temperature of at least 55°C and hydrolyzing the reaction mixture.

2. The process according to claim 1, wherein the temperature in the reaction step is not higher than 100°C.

3. The process according to claim 1, wherein said hydrocarbon is an aromatic hydrocarbon.

4. The process according to claim 1, wherein said hydrocarbon is toluene.

5. The process according to claim 1, wherein said hydrocarbon is used in an amount of 1 to 50 parts by weight per one part by weight of 2-methylfuran.

6. The process according to claim 1, wherein the reaction is carried out by feeding 2-methylfuran, phosgene and N,N-dimethylformamide into a reaction system concurrently.

## Patentansprüche

1. Verfahren zur Herstellung von 5-Methyl-2-furfural, umfassend die Schritte des Umsetzens von 2-Methylfuran, Phosgen und N,N-Dimethylformamid in einem Reaktionsmedium, umfassend einen Kohlenwasserstoff, bei einer Temperatur von mindestens 55°C und des Hydrolysierens des Reaktionsgemisches.

2. Verfahren gemäß Anspruch 1, wobei die Temperatur in dem Umsetzungsschritt nicht höher als 100°C ist.

3. Verfahren gemäß Anspruch 1, wobei der Kohlenwasserstoff ein aromatischer Kohlenwasserstoff ist.

4. Verfahren gemäß Anspruch 1, wobei der Kohlenwasserstoff Toluol ist.

5. Verfahren gemäß Anspruch 1, wobei der Kohlenwasserstoff in einer Menge von 1 bis 50 Gewichtsteilen pro 1 Gewichtsteil an 2-Methylfuran verwendet wird.

6. Verfahren gemäß Anspruch 1, wobei die Umsetzung durch gleichzeitiges Einleiten von 2-Methylfuran, Phosgen und N,N-Dimethylformamid in ein Reaktionssystem durchgeführt wird.

## Revendications

1. Procédé pour la préparation de 5-méthyl-2-furfural comprenant les étapes de réaction de 2-méthylfuranne, de phosgène et de N,N-diméthylformamide dans un milieu réactionnel comprenant un hydrocarbure à une température d'au moins 55°C et d'hydrolyse du mélange réactionnel.

2. Procédé selon la revendication 1, dans lequel la température dans l'étape de réaction est d'au plus 100°C.

3. Procédé selon la revendication 1, dans lequel ledit hydrocarbure est un hydrocarbure aromatique.

4. Procédé selon la revendication 1, dans lequel ledit hydrocarbure est le toluène.

5. Procédé selon la revendication 1, dans lequel ledit hydrocarbure est utilisé dans une quantité de 1 à 50 parties en poids pour une partie en poids de 2-méthylfuranne.

6. Procédé selon la revendication 1, dans lequel la réaction est réalisée en introduisant du 2-méthylfuranne, du phosgène et du N,N-diméthylformamide concurremment dans un système réactionnel.
